(19) Europäisches Patentamt
European Patent Office
Office européen des brevets

(11) Veröffentlichungsnummer: **0 269 011**
**A1**

(12) **EUROPÄISCHE PATENTANMELDUNG**

(21) Anmeldenummer: **87117138.5**

(22) Anmeldetag: **20.11.87**

(51) Int. Cl.⁴: **C07C 45/50** , C07C 47/02

(30) Priorität: **27.11.86 DE 3640615**

(43) Veröffentlichungstag der Anmeldung:
**01.06.88 Patentblatt 88/22**

(84) Benannte Vertragsstaaten:
**AT BE CH DE ES FR GB IT LI LU NL SE**

(71) Anmelder: **Ruhrchemie Aktiengesellschaft**
**Bruchstrasse 219**
**D-4200 Oberhausen 13(DE)**

(72) Erfinder: **Bach, Hanswilhelm, Dr. Dipl.-Chem.**
**Alleestrasse 56**
**D-4100 Duisburg 11(DE)**
Erfinder: **Cornils, Boy, Dr. Dipl.-Chem.**
**Kirschgartenstrasse 6**
**D-6238 Hofheim(DE)**
Erfinder: **Gick, Wilhelm, Dr. Dipl.-Chem.**
**Im Buschhuck 8**
**D-4100 Duisburg 74(DE)**
Erfinder: **Diekhaus, Gerhard, Dr. Dipl.-Chem.**
**Walsumermarkstrasse 89**
**D-4200 Oberhausen 11(DE)**
Erfinder: **Konkol, Werner, Dr. Dipl.-Chem.**
**Lützowstrasse 40 a**
**D-4200 Oberhausen 11(DE)**
Erfinder: **Wiebus, Ernst**
**Ferdinandstrasse 77**
**D-4200 Oberhausen 11(DE)**

(74) Vertreter: **Reichelt, Karl-Heinz, Dr.**
**Ruhrchemie Aktiengesellschaft Postfach 13 01 60**
**D-4200 Oberhausen 11(DE)**

(54) **Verfahren zur Herstellung von Aldehyden.**

(57) Die Erfindung betrifft ein Verfahren zur Herstellung von Aldehyden durch Umsetzung von Olefinen mit Kohlenmonoxid und Wasserstoff in Gegenwart wasserlöslicher Rhodiumkomplexkatalysatoren. Hierbei wird zusammen mit dem Reaktionsprodukt ein Teil der wäßrigen Lösung des Katalysatorsystems abgezogen und durch frische Lösung ersetzt.

EP 0 269 011 A1

# Verfahren zur Herstellung von Aldehyden

Die vorliegende Erfindung betrifft ein Verfahren zur Herstellung von Aldehyden durch Hydroformylierung olefinischer Verbindungen in Gegenwart wasserlöslicher Rhodium-Komplexverbindungen als Katalysator. Ziel der neuen Arbeitsweise ist die Aufrechterhaltung der ursprünglichen selektiven Wirksamkeit des Katalysatorsystems hinsichtlich der Bildung von n-Aldehyden durch absatzweise oder kontinuierliche Entfernung von Abbau-und Umwandlungsprodukten der Komplexliganden.

Aus der DE 26 27 354 C3 ist ein Hydroformylierungsverfahren bekannt, bei dem Olefin, Kohlenmonoxid und Wasserstoff in flüssiger Phase in Gegenwart von Wasser und wasserlöslichen Rhodium-Komplexverbindungen umgesetzt werden. Die Löslichkeit der katalytisch aktiven Rhodium-Komplexverbindungen wird durch Verwendung sulfonierter Triarylphosphine als Komplexbestandteil erreicht. Diese Arbeitsweise hat eine Reihe bemerkenswerter Vorteile. Sie erlaubt insbesondere eine sehr einfache Trennung von Reaktionsprodukt und Katalysator und stellt eine annähernd vollständige Rückgewinnung des Rhodiums sicher. Die Abscheidung des Katalysators vom Reaktionsprodukt erfolgt einfach durch Trennung von wäßriger und organischer Phase, d.h. ohne Destillation und damit ohne thermische Belastung der gebildeten Aldehyde und Alkohole. Aufgrund der überaus geringen Löslichkeit des Katalysators in Aldehyd und Alkohol wird mit dem Reaktionsprodukt auch kaum Edelmetall ausgetragen. Schließlich wird auch eine Vergiftung des Katalysators durch hochsiedende Nebenprodukte - sie entstehen z.B. durch Aldolisierung bxw. Aldolkondensation oder Acetalbilding - weitgehend vermieden.

Die als Katalysator eingesetzten Rhodium-Komplexverbindungen enthalten je Rhodiumatom maximal 3 Phosphinmoleküle. Sie entsprechen der allgemeinen Form $HRh(CO)_xL_{4-x}$, wobei L für den wasserlöslichen Phosphinliganden steht und x die Zahlen 1 bis 3 bedeutet.

Um die Stabilität der Rhodium-Komplexverbindungen zu erhöhten wendet man sie in Form eines Katalysatorsystems an, das, bezogen auf vorhandenes Rhodium, einen hohen Phosphinüberschuß aufweist. Üblicherweise setzt man auf 1 g-Atom Rhodium 10 bis 300 Mol, bevorzugt 50 bis 150 Mol, wasserlösliches Phosphin ein.

Die wasserlöslichen Phosphinliganden folgen der allgemeinen Formel

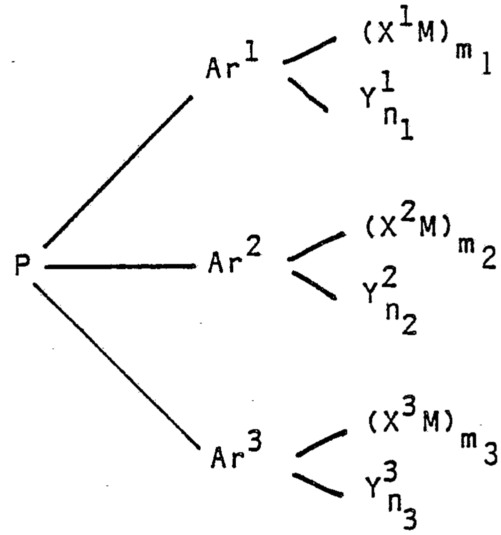

In ihr bedeuten, $Ar^1$, $Ar^2$, $Ar^3$ jeweils eine Phenyl-oder Naphthylgruppe, $Y^1$, $Y^2$, $Y^3$ jeweils eine geradkettige oder verzweigte Alkylgruppe mit 1 bis 4 C-Atomen, eine Alkoxygruppe, ein Halogenatom, die OH-, CN-, $NO_2$-oder $R^1R^2N$-Gruppen, in der $R^1$ und $R^2$ für jeweils eine geradkettige oder verzweigte Alkylgruppe mit 1 bis 4 C-Atomen stehen; $X^1$, $X^2$, $X^3$ ist jeweils ein Carboxylat-($COO^-$-) und/oder ein Sulfonat-($(S)_3^-$-)Rest, $m_1$, $m_2$, $m_3$ sind gleiche oder verschiedene ganze Zahlen von 0 bis 3, wobei mindestens eine Zahl $m_1$, $m_2$ oder $m_3$ gleich oder größer 1 ist; $n_1$, $n_2$, $n_3$ sind gleiche oder verschiedene ganze Zahlen von 0 bis 5, M ist ein Alkalimetallion, ein Äquivalent eines Erdalkalimetall-oder Zinkions, ein Ammonium-oder quaternäres Ammoniumion der allgemeinen Formel $N(R^3R^4R^5R^6)^+$, in der $R^3$, $R^4$, $R^5$, $R^6$ jeweils für eine geradkettige oder verzweigte Alkylgruppe mit 1 bis 18 C-Atomen oder eine Aralkylgruppe mit 7 bis 14 C-Atomen steht. Bewährt haben sich insbesondere quaternäre Ammoniumgruppen, in denen drei der Reste $R^3$, $R^4$, $R^5$, $R^6$ jeweils 1 bis 4 Kohlenstoffatome enthalten und der vierte Rest eine Aralkylgruppe mit 7 bis 14 C-Atomen ist.

Bevorzugt werden wasserlösliche Triarylphosphine der vorstehend beschriebenen allgemeinen Formel, in der $Ar^1$, $Ar^2$, $Ar^3$ jeweils einen Phenylrest und $X^1$, $X^2$, $X^3$ jeweils einen Sulfonatrest oder einen Carboxylatrest bedeuten. Beispiele für Verbindungen der oben wiedergegebenen allgemeinen Formel sind Triphenylphosphin-tri-Na-trisulfonat, Triphenylphosphin-tri-(tetraalkylammonium)-trisulfonat, Triphenylphosphin-tri-Na-tricarboxylat.

Die sulfonierten oder carboxylierten Arylphosphine können als einheitliche Verbindungen eingesetzt werden. Es können aber auch Gemische aus

Phosphinen verwendet werden, die unterschiedlich viele Sulfonatgruppen oder Carboxylatgruppen enthalten, also z.B. Gemische aus Salzen der Triarylphosphintrisulfonsäuren und Triarylphosphindisulfonsäuren. Darüber hinaus müssen die Sulfonate oder Carboxylate nicht dasselbe Kation enthalten. Auch Gemische aus Salzen, die sich von unterschiedlichen Metallen ableiten und/oder Ammonium-und/oder quaternäre Alkylammoniumionen enthalten, sind geeignet.

Das aus Rhodium-Komplexverbindung und wasserlöslichen Phosphinen im Überschuß bestehende Katalysatorsystem wird als wäßrige Lösung eingesetzt, die im allgemeinen 50 bis 800 Gew.-ppm Rhodium und 25 bis 30 Gew.-% wasserlösliches Phosphin, jeweils bezogen auf die Lösung, enthält. Besonders bewährt hat es sich mit Lösungen zu arbeiten, die 50 bis 800 Gew.-ppm, vorzugsweise 200 bis 600 Gew.-ppm, Rhodium und 26 bis 28 Gew.-% wasserlösliches Phosphin enthalten.

Ein besonderer Vorteil des vorstehend beschriebenen Katalysatorsystems ist, daß es sehr selektiv die Bildung des für die Weiterverwendung meist wertvolleren n-Aldehyds bewirkt, während iso-Aldehyd nur in ganz untergeordneter Menge entsteht. So erhält man z.B. bei der Hydroformylierung von Propylen neben etwa 95 Gew.-% n-Butyraldehyd nur 5 % der iso-Verbindung.

Bei wiederholtem oder kontinuierlichem Einsatz derselben Katalysatorlösung nimmt im Laufe der Zeit die selektive Wirkung des Katalysatorsystems ab. Unter unveränderten Reaktionsbedingungen verringert sich dann die je Mengeneinheit Rhodium und je Zeiteinheit gebildete Menge n-Aldehyd zugunsten der Entstehung von iso-Aldehyd. Eine Ursache dieses Selektivitätsverlustes ist die Umwandlung der wasserlöslichen Phosphine in Verbindungen, die zur Komplexbildung mit Rhodium nicht mehr fähig sind. So werden durch Hydrolyse die P-C-Bindungen zwischen dem Phosphoratom und den Sulfonat-oder Carboxylatgruppen enthaltenden Phenyl-oder Naphthylresten aufgebrochen oder durch Oxidation Phosphor(V)-Verbindungen gebildet. Neben anderen, nicht identifizierten Substanzen wurden als Umwandlungsprodukte von Salzen der Triphenylphosphinsulfonsäuren, z.B. Salze aromatischer Sulfonsäuren und der Disulfophenylphosphinsäure gefunden. Diese Reaktionen haben zur Folge, daß sich das Verhältnis der Anzahl Phosphinmoleküle zur Anzahl Rhodiumatome verringert, wodurch die selektive Wirkung des Katalysatorsystems herabgesetzt wird.

Da die Wirtschaftlichkeit katalytischer Prozesse in vielen Fällen sehr stark von der Lebensdauer des Katalysators abhängt, ist man bestrebt, die Aktivität des Katalysators und seine selektive Wirksamkeit über möglichst lange Zeit auf einem gleich hohen Niveau zu halten.

Es bestand daher die Aufgabe ein Verfahren zu entwickeln, das die durch Umwandlung der wasserlöslichen Phosphine verursachte Selektivitätsminderung des Katalysatorsystems vermeidet oder zumindest in Grenzen hält.

Die Erfindung besteht in einem Verfahren zur Herstellung von Aldehyden durch Umsetzung aliphatischer Olefine mit 2 bis 12 Kohlenstoffatomen mit Kohlenmonoxid und Wasserstoff in flüssiger Phase und in Gegenwart einer wäßrigen Lösung, die 50 bis 800 Gew.-ppm Rhodium und 25 bis 30 Gew.-% Phosphine, jeweils bezogen auf die wäßrige Lösung als Katalysatorsystem und daneben Umwandlungsprodukte der Phosphine enthält. Es ist dadurch gekennzeichnet, daß zusammen mit dem Reaktionsprodukt ein Teil der wäßrigen Lösung des Katalysatorsystems abgezogen und durch frische Lösung ersetzt wird.

Durch die ständige Erneuerung eines Teils der Lösung des Katalysatorsystems wird sichergestellt, daß die Konzentration der Umwandlungsprodukte einen bestimmten Grenzwert nicht überschreitet.

Überraschenderweise hat sich nämlich herausgestellt, daß mit steigender Konzentration der Umwandlungsprodukte die Geschwindigkeit ihrer Bildung in der Katalysatorlösung stark zunimmt. Möglicherweise begünstigen die Umwandlungsprodukte ihre eigene Entstehung aus den wasserlöslichen Phosphinen, so daß man von einer Art autokatalytischem Prozeß sprechen kann. Es ist aber auch denkbar, daß durch die Verarmung der Lösung an Phosphorliganden Rhodium-Komplexverbindungen dissoziieren und Rhodiumatome unter Ausbildung von Metall-Metall-Bindungen zu Clustern zusammentreten, die die Bildung von Phosphin-Umwandlungsprodukten katalytisch beschleunigen. Dieses Verhalten führt dazu, daß die Phosphinkonzentration im Laufe der Zeit nicht linear, sondern überproportional abnimmt.

Geringe Mengen Umwandlungsprodukte in der Katalysatorlösung stören nicht. Welche Mengen tolerierbar sind, kann nicht allgemein gültig gesagt werden, sondern ist individuell u.a. von den Reaktionsbedingungen und den eingesetzten olefinischen Verbindungen abhängig. Maßgebend für die Anwendung des Verfahrens der Erfindung ist die Zunahme der iso-Verbindung im Reaktionsgemisch verglichen mit dem iso-Aldehyd-Anteil im Reaktionsprodukt bei Einsatz eines frischen Katalysators.

Unter dem Begriff Umwandlungsprodukte im Sinne der vorliegenden Erfindung versteht man alle aus den wasserlöslichen Phosphinen während der Hydroformylierungsreaktion entstehenden Phosphorverbindungen, die mit Rhodium keine Komplexe bilden.

Die Behandlung der wäßrigen Lösung des

Katalysatorsystems nach dem neuen Verfahren kann kontinuierlich erfolgen. Dann wird zusammen mit dem Reaktionsprodukt ständig ein Anteil der Katalysatorlösung dem Reaktionsgemisch entnommen und durch frische Lösung ersetzt. Es ist aber auch möglich, diskontinuierlich von Zeit zu Zeit Lösungsanteile auszuschleusen und durch frische Lösung zu ersetzen.

Der Katalysator kann dem Reaktionsgemisch im formierten Zustand, d.h. als Phosphin und Kohlenmonoxid enthaltende Rhodium-Komplexverbindung zugeführt werden. Mit gleich gutem Erfolg kann man aber auch die Bestandteile der Rhodium-Komplexverbindung zudosieren: das Rhodium als in Wasser lösliches Rhodiumsalz wie Rhodiumsulfat, Rhodiumacetat, Rhodium-2-ethylhexanoat und das carboxylierte oder sulfonierte Triphenyl-oder Trinaphthylphosphin z.B. als Natriumsalz. Beide Komplexbestandteile können auch getrennt als Lösungen eingesetzt werden. Die Bildung der Komplexverbindung erfolgt dann unter den Bedingungen der Hydroformylierungsreaktion und unter Mitwirkung des im Synthesegas enthaltenen Kohlenmonoxids. Die Konzentration von Rhodium und Phosphin in der frischen Lösung stimmt im allgemeinen mit der Konzentration dieser Bestandteile in der Katalysatorlösung überein, liegt also im Bereich von 50 bis 800 Gew.-ppm Rhodium und 25 bis 30 Gew.-% Phosphin.

Die abgetrennte Katalysatorlösung wird zur Rückgewinnung der Rhodium-Komplexverbindung und überschüssiger wasserlöslicher Phosphine nach bekannten Verfahren aufgearbeitet. Geeignet ist z.B. die Extraktion der zuvor angesäuerten Lösung mit der Lösung eines Amins in einem organischen Lösungsmittel und anschließende Behandlung der organischen Phase mit der wäßrigen Lösung einer anorganischen Base. Nach einer anderen Arbeitsweise trennt man Rhodium-Komplexverbindung und Phosphine einschließlich ihrer Umwandlungsprodukte nach einem Membrantrennverfahren. Die Rhodium-Komplexverbindung kann unmittelbar wieder als Katalysatorbestandteil eingesetzt werden, während das Phosphin durch Extraktion mit Amin wiedergewonnen wird.

Nach dem erfindungsgemäßen Verfahren lassen sich Olefine mit 2 bis 12 Kohlenstoffatomen hydroformylieren. Diese Olefine können linear oder verzweigt sein und eine endständige oder innenständige Doppelbindung enthalten. Ebenso können Cycloolefine mit 6 bis 12 Kohlenstoffatomen umgesetzt werden. Beispiele für die vorstehend beschriebenen Olefine sind: Ethylen, Propylen, i-Buten, 2-Buten, 1-Penten, 2-Methyl-1-buten, 4,4-Dimethyl-1-nonen, 1-Dode cen, Cyclohexen, Dicyclopentadien. Bevorzugt werden lineare Olefine mit 2 bis 8 Kohlenstoffatomen wie Ethylen, Propylen, 1-Buten, 1-Penten, 1-Hexen, 1-Hepten und 1-Octen und als Cycloolefin Dicyclopentadien eingesetzt.

Der Gesamtdruck von Wasserstoff und Kohlenmonoxid beträgt 0,1 bis 20 MPa, vorzugsweise 1 bis 10 MPa. Die Zusammensetzung des Synthesegases, d.h. das Verhältnis von Kohlenmonoxid zu Wasserstoff kann in weiten Grenzen variiert werden. Im allgemeinen setzt man Synthesegas ein, in dem das Volumverhältnis von Kohlenmonoxid zu Wasserstoff 1 : 1 beträgt oder von diesem Wert nur wenig abweicht. Die Umsetzung erfolgt bei Temperaturen von 20 bis 150°C, sie kann sowohl kontinuierlich als auch absatzweise durchgeführt werden.

Das Verfahren wird durch die folgenden Beispiele verdeutlicht.

Beispiel 1 (Vergleichsbeispiel)

In einer wäßrigen Katalysatorlösung, die 26 Gew.-% eines Gemisches der Natriumsalze der Triphenylphosphin-trisulfonsäure und der Triphenylphosphin-disulfonsäure sowie 450 ppm Rh enthält, werden unter Rühren bei einer Temperatur von 125°C und einem Druck von 5 MPa Propylen, Kohlenmonoxid und Wasserstoff im Volumverhältnis 1 : 1 : 1 zur Reaktion gebracht. Je Liter Katalysatorlösung und Stunde werden 2,1 mole eines Gemisches aus 95 Gew.-% n-und 5 Gew.-% iso-Butyraldehyd gebildet. Mit zunehmender Laufzeit steigt der Anteil iso-Aldehyd im Reaktionsprodukt stetig an, d.h. die Selektivität der Umsetzung geht zurück.

Beispiel 2

Beispiel 1 wird mit dem Unterschied wiederholt, daß bei Abnahme der Selektivität der Umsetzung, sie zeigt sich in einer Erhöhung des iso-Aldehyd-Anteils im Reaktionsprodukt von 5 auf 7 Gew.-%, 10 % der Katalysatorlösung abgelassen und durch die gleiche Menge Frischlösung (mit 26 Gew.-% und 450 Gew.-ppm Rhodium als Acetat) ersetzt werden. Auf diesem Wege gelingt es, die ursprüngliche Selektivität der Reaktion wiederherzustellen. Der ausgeschleuste Katalysator wird zur Rückgewinnung von Rhodium und Phosphin z.B. durch Extraktion aufgearbeitet. Der Vorgang kann beliebig oft wiederholt werden, so daß über einen langen Zeitraum ein gleichbleibender Reaktionsablauf sichergestellt wird.

**Ansprüche**

1.) Verfahren zur Herstellung von Aldehyden durch Umsetzung aliphatischer Olefine mit 2 bis 12 Kohlenstoffatomen mit Kohlenmonoxid und Wasserstoff bei 20 bis 150°C und 0,1 bis 20 MPa in flüssiger Phase und in Gegenwart einer wäßrigen Lösung, die 50 bis 800 Gew.-ppm Rhodium und 25 bis 30 Gew.-% Phosphine, jeweils bezogen auf die wäßrige Lösung, als Katalysatorsystem und daneben Umwandlungsprodukte der Phosphine enthält, dadurch gekennzeichnet, daß zusammen mit dem Reaktionsprodukt ein Teil der wäßrigen Lösung des Katalysatorsystems abgezogen und durch frische Lösung ersetzt wird.

2.) Verfahren nach Anspruch 1, dadurch gekennzeichnet, daß die frische Lösung Rhodium als wasserlösliches Salz und wasserlösliches Phosphin enthält und die Bildung des Katalysatorsystems in der Hydroformylierungszone erfolgt.

Europäisches Patentamt

# EUROPÄISCHER RECHERCHENBERICHT

Nummer der Anmeldung

EP 87117138.5

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| D,A | DE - B2 - 2 627 354 (RHONE-POULENC)<br>* Anspruch 1 *<br>-- | 1 | C 07 C 45/50<br>C 07 C 47/02 |
| A | DE - A1 - 3 341 035 (RUHRCHEMIE)<br>* Anspruch 1 *<br>-- | 1 | |
| A | DE - A1 - 3 511 428 (RUHRCHEMIE)<br>* Anspruch 1 *<br>-- | 1 | |
| P,A | EP - A1 - 0 216 315 (RUHRCHEMIE)<br>* Ansprüche 1,11 *<br>-- | 1 | |
| A | WO - A1 - 82/03 856 (EASTMAN KODAK)<br>* Ansprüche 1,3 *<br>-- | 1 | **RECHERCHIERTE SACHGEBIETE (Int. Cl.4)** |
| A | US - A - 4 547 595 (BIAU-HUNG CHANG)<br>* Zusammenfassung *<br>-- | 1 | C 07 C 45/00<br>C 07 C 47/00<br>B 01 J 31/00 |
| A | DE - A1 - 3 017 651 (MITSUBISHI CHEMICAL)<br>* Anspruch 1 *<br>---- | 1 | |

Der vorliegende Recherchenbericht wurde für alle Patentansprüche erstellt.

| Recherchenort | Abschlußdatum der Recherche | Prüfer |
|---|---|---|
| WIEN | 29-02-1988 | REIF |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82